# EUROPÄISCHE PATENTANMELDUNG

(11) **EP 0 570 843 A2**
(43) Veröffentlichungstag der Anmeldung: **24.11.1993**
(21) Anmeldenummer: 93107791.1
(22) Anmeldetag: 13.05.1993
(51) Int. Cl.: C07D 201/12, C08J 11/14, C07D 201/16

(54) **Verfahren zur Rückgewinnung von Caprolactam aus Oligomeren und/oder Polymeren des Caprolactams**

(30) Priorität: 21.05.1992 DE 4216848
(71) Anmelder: BASF Aktiengesellschaft, D-67063 Ludwigshafen (DE)
(72) Erfinder: Fuchs, Hugo, Dr., W-6700 Ludwigshafen (DE); Priester, Claus-Ulrich, Dr., W-6700 Ludwigshafen (DE); Neubauer, Gerald, Dr., W-6940 Weinheim (DE); Brunner, Erwin, Dr., W-6940 Weinheim (DE); Ritz, Josef, Dr., W-6700 Ludwigshafen (DE); Kopietz, Michael, Dr., W-6718 Gruenstadt (DE)

(57) **Zusammenfassung**

Verfahren zur Rückgewinnung von Caprolactam aus Oligomeren und/oder Polymeren des Caprolactams, welches folgende Schritte umfaßt:
a) Behandlung von Oligomeren und/oder Polymeren des Caprolactams mit 1 bis 20 Gew.-Teilen Wasser je Gew.-Teil Oligo- und/oder Polymer bei einer Temperatur von 200 bis 350°C unter erhöhtem Druck mit einer Verweilzeit von 0,5 bis 10 Stunden unter Bildung eines wäßrigen Reaktionsgemisches, das Polycaprolactam, monomeres Caprolactam und dessen Oligomere enthält und
b) Leiten des in a) erhaltenen wäßrigen Reaktionsgemisches in eine Aluminiumoxid-Wirbelschicht bei einer Temperatur von 250 bis 400°C unter Erhalt eines Gemisches aus Wasserdampf und Caprolactam.

## Beschreibung

Gegenstand der Erfindung ist ein Verfahren zur Rückgewinnung von Caprolactam aus Oligomeren und/oder Polymeren des Caprolactams durch hydrolytische Spaltung von Oligomeren und/oder Polymeren des Caprolactams mit Wasser.

Bei der Herstellung von Polycaprolactam und dessen Verarbeitung durch Verformen aus der Schmelze, z.B. Spinnen zu Fäden, Extrudieren zu Folien oder Formteilen durch Spritzgießen fällt Polyamidabfall an, der entsorgt werden muß. Ferner sind auch Polyamidteile, z.B. Spritzguß- oder Extrusionsteile, Folien, Verpackungen oder Gewebe nach ihrem Gebrauch entsorgungsbedürftig. Es bietet sich deshalb an, entsorgungsbedürftige Polyamide wiederum unter Gewinnung von Caprolactam aufzuarbeiten.

Aus der US-PS 4,605,762 ist ein Verfahren zur hydrolytischen Spaltung von Polykondensaten bekannt, bei dem man Polykondensate wie Polyester, Polyamid-6,6 oder Polycaprolactam unter Mitverwendung der 2- bis 20-fachen Menge Wasser bei einer Temperatur von 200 bis 300°C unter erhöhtem Druck spaltet. Da die Spaltung nicht vollständig erfolgt, verbleibt immer ein erheblicher Rückstand an Oligomeren und nicht gespaltenem Polycaprolactam. Darüber hinaus gibt es in dem in der US-PS beschriebenen Verfahren keinen Hinweis, wie die Qualität des erzeugten monomeren Caprolactam verbessert werden kann, so daß es ohne Nachteil in die Reinigungsstufe bei der Caprolactamherstellung eingeschleust werden kann.

Aus der EP-A 46 183 ist auch schon bekannt, Oligomere des Caprolactam, die bei der Extraktion von Polycaprolactam anfallen, in einer Wirbelschicht aus Aluminiumoxid bei erhöhter Temperatur unter Mitverwendung von Wasser zu monomeren Caprolactamen zu spalten. Es wird jedoch kein Hinweis gegeben, wie zu verfahren ist, wenn neben Oligomeren des Caprolactam zusätzlich Polycaprolactam in der wäßrigen Suspension enthalten ist, zumal das Einbringen von Polymeren in eine Wirbelschicht zu einem Verkleben der Wirbelschicht führen kann.

Es war deshalb die technische Aufgabe gestellt, ein Verfahren zur Rückgewinnung von Caprolactam aus Oligomeren und/oder Polymeren des Caprolactams zur Verfügung zu stellen, das mit hohen Ausbeuten verläuft und bei dem praktisch keine Rückstände anfallen und zudem ein Caprolactam mit verbesserter Qualität erhalten wird, das ohne Nachteil in die Reinigungsstufe der Caprolactamherstellung eingeführt werden kann.

Diese technische Aufgabe wird gelöst in einem Verfahren zur Rückgewinnung von Caprolactam aus Oligomeren und/oder Polymeren des Caprolactams, welches folgende Schritte umfaßt:
Behandlung von Oligomeren und/oder Polymeren des Caprolactams mit 1 bis 20 Gew.-Teilen Wasser je Gew.-Teil Oligo- bzw. Polymer bei einer Temperatur von 200 bis 350°C unter erhöhtem Druck mit einer Verweilzeit von 0,5 bis 10 Stunden unter Bildung eines wäßrigen Reaktionsgemisches, das Polycaprolactam, monomeres Caprolactam und dessen Oligomere enthält und anschließend
Leiten des in A) erhaltenen wäßrigen Reaktionsgemisches in eine Aluminiumoxid-Wirbelschicht bei einer Temperatur von 250 bis 400°C, bevorzugt von 270 bis 400°C unter Erhalt eines Gemisches aus Wasserdampf und monomerem Caprolactam.

Das neue Verfahren hat den Vorteil, daß es mit hohen Caprolactamausbeuten verläuft und de facto keine entsorgungsbedürftigen Rückstände anfallen. Ferner hat das neue Verfahren den Vorteil, daß es unschwer kontinuierlich im technischen Maßstab durchführbar ist. Ferner hat das neue Verfahren den Vorteil, daß die Qualität des erhaltenen Caprolactam ein Einbringen in den Reinigungsschritt bei der Caprolactamherstellung zuläßt.

Erfindungsgemäß geht man von Polycaprolactam aus, das entsorgt werden soll, z.B. Abfällen, die bei der Herstellung von Polycaprolactam oder dessen Verarbeitung aus der Schmelze, z.B. bei der Herstellung von Fäden oder der Herstellung von extrudierten Teilen oder spritzgegossenen Formteilen, ferner bei der Herstellung von Folien anfällt. Geeignet sind auch Polycaprolactam-Formteile, die Extrudate, Spritzgußteile oder Gewebe, nach deren Gebrauch diese entsorgungsbedürftig sind. Vorteilhaft hat das zu spaltende Polycaprolactam eine Teilchengröße von 1 bis 100 mm. Dies erreicht man z.B. durch Mahlen von Formteilen, die gegebenenfalls vorher unter Druck und Wärme verdichtet werden.

Bei der Rückgewinnung von Caprolactam aus Oligomeren oder Oligomeren und Polymeren des Caprolactams, kann man prinzipiell alle Arten von Oligomeren des Caprolactams einsetzen. Üblicherweise setzt man Oligomere ein, die bei der Polykondensation von Caprolactam entstehen, da bei dieser Reaktion in der Regel ein Gleichgewicht mit üblicherweise etwa 90 Gew.-% Polymere und etwa 10 Gew.-% Caprolactam und Oligomere vorliegt. Die Oligomere, die sich im allgemeinen aus etwa 50 Gew.-% Dimeren und Trimeren und zu etwa 50 Gew.-% aus Tetrameren und höheren Oligomeren zusammensetzen, gewinnt man im allgemeinen zusammen mit Caprolactam durch Extraktion mit Wasser aus dem vorgenannten Reaktionsgemisch.

In einer Stufe a) werden die Oligo- bzw. Polymeren des Caprolactams zunächst mit 1 bis 20 Gew.-Teilen Wasser, insbesondere 2 bis 10 Gew.-Teilen Wasser je Gew.-Teil Oligo- bzw. Polymer hydrolytisch gespalten. Hierbei hält man eine Temperatur von 200 bis 350°C, insbesondere von 250 bis 300°C ein. Die Behandlung erfolgt unter erhöhtem Druck, vorteilhaft bei 15 bis 200 bar, wobei der erhöhte Druck zusätzlich durch Aufpressen von Inertgas wie Stickstoff erzeugt wird. Es versteht sich, daß eine flüssige Wasserphase aufrechterhalten wird. Die Behandlung erfolgt mit einer Verweilzeit von 0,5 bis 10 Stunden, insbesondere 1 bis 5 Stunden. Ferner hat es sich bewährt, zusätzlich Phosphorsäure oder Alkalimetallhydroxide, insbesondere Natriumhydroxid in einer Menge von 0,001 bis 0,1 Gew.-Teilen je Gew.-Teil Oligo- bzw. Polymer des Caprolactams mitzuverwenden.

Man erhält ein wäßriges Reaktionsgemisch, das Polycaprolactam, monomeres Caprolactam und dessen Oligomere enthält. Eine typische Zusammensetzung ist beispielsweise 1 bis 70 Gew.-% monomeres Caprolactam, 0,1 bis 10 Gew.-% Oligomere des Caprolactams sowie 1 bis 99 Gew.-% suspendiertes Polycaprolactam, bezogen auf eingesetztes Oligo- und/oder Polymer des Caprolactams.

Das so erhaltene Reaktionsgemisch aus der Stufe a) wird in Stufe b) in eine Aluminiumoxid-Wirbelschicht bei einer Temperatur von 250 bis 400°C, bevorzugt von 270 bis 400°C geleitet und ein Gemisch aus Wasserdampf und Caprolactam erhalten.

Das Reaktionsgemisch aus der Stufe a) kann vor Einführen in die Wirbelschicht entspannt werden, zweckmäßig führt man jedoch das Reaktionsgemisch über eine Düsenöffnung direkt unter Entspannen in die Wirbelschicht der Stufe b) ein. Das Einbringen in die Wirbelschicht kann auch durch Einblasen mittels einer mit einem Inertgas betriebenen Düse erfolgen.

Als Aluminiumoxid eignen sich die verschiedenen Modifikationen wie Tonerde, **α**- oder **γ**-Aluminiumoxid. Besonders bewährt hat sich γ-Aluminiumoxid als Katalysator. Der Katalysator wird mit Inertgas wie Kohlendioxid, Argon oder Stickstoff, vorzugsweise Stickstoff, in wirbelnder Bewegung gehalten. Zweckmäßig verwendet man Aluminiumoxid in Korngrößen von 0,05 bis 1,5 mm, insbesondere 0,1 bis 0,4 mm. Die Höhe der Wirbelschicht wird vorteilhaft so gewählt, daß die Verweilzeit des Oligomeren und polymeren Caprolactam in der Katalysatorschicht von 0,1 bis 30, insbesondere von 0,5 bis 10 Sekunden betragen. Vorteilhaft führt man die Behandlung in der Wirbelschicht bei Normaldruck durch. Es kann jedoch auch schwach verminderter oder leicht überhöhter Druck, z.B. von 0,5 bis zu 2 bar angewandt werden.

Vorteilhaft hält man in der Wirbelschicht eine Temperatur von 290 bis 360°C ein. Es ist deshalb auch zweckmäßig, das Inertgas mit einer Temperatur von 290 bis 400°C der Wirbelschicht zuzuführen.

Aus dem aus der Wirbelschicht austretenden Gasgemisch werden die kondensierbaren Anteile durch Kondensieren abgeschieden und dann z.B. durch Destillation aufgearbeitet. Es ist auch möglich, die anfallende wäßrige Lösung direkt in die Reinigungsstufe bei der Caprolactamherstellung einzuleiten.

Das Verfahren nach der Erfindung sei an folgenden Beispielen veranschaulicht.

### Beispiel 1

In einen Autoklaven wurden 300 g Polyamid 6 und 1.000 ml Wasser eingefüllt, mit Stickstoff gespült, 5 bar Stickstoff aufgepreßt und auf 250°C aufgeheizt. Der Gesamtdruck betrug ca. 60 bar.

Nach 5 Stunden wurde der Autoklaveninhalt abgekühlt.

Das gesamte Produkt wurde langsam in einen Wirbelofen, der mit 600 g Aluminiumoxid, Typ Puralox (Puralox NX a-150, Condea Chemie, Schüttgewicht 700-900 g/l, Korngrößenverteilung < 100 µm, max. 5 %; > 500 µm, max. 2 %), gefüllt war, eindosiert. Die Wirbelschicht wurde mit Stickstoff am Wirbeln gehalten. Die Temperatur in der Wirbelschicht betrug 300°C, die Verweilzeit ca. 2 Sekunden. Das die Wirbelschicht verlassende Dampfgemisch wurde kondensiert.

Im Kondensat wurden 292 g Caprolactam und 4 g Oligomere/Polymere gefunden. Die Ausbeute an Caprolactam betrug 97,3 %, bezogen auf das eingesetzte Polyamid 6.

### Beispiel 2

100 g Oligomere des Caprolactams (ca. 50 Gew.-% Dimere und Trimere, Rest: Tetramere und höhere Oligomere) wurden in 100 g Wasser suspendiert und in einen 500 ml Autoklaven gefüllt. Der Autoklav wurde mit Stickstoff gespült und anschließend auf 200°C erhitzt. Nach 30 Minuten wurde der Autoklaveninhalt bei 200°C unter einem Druck von ca. 15 bar in die Wirbelschicht eines Wirbelofens entspannt. Der Wirbelofen war mit 600 g Al₂O₃ (Puralox; s. Beispiel 1) gefüllt. Die Wirbelschicht wurde durch Einblasen von Stickstoff am Wirbel gehalten. Die Temperatur in die Wirbelschicht wurde bei 300°C gehalten. Das die Wirbelschicht verlassende Dämpfegemisch wurde kondensiert. Nach Abdestillieren des Wassers erhielt man 97 g Caprolactam.

### Beispiel 3

300 g Polyamid 6 (rel. Viskosität 2,7; gemessen bei einer Konzentration von 1 g pro 100 ml in 96 gew.-%iger Schwefelsäure bei 25°C) wurde zusammen mit 700 g Wasser in einen 2 l-Autoklaven gefüllt und mit Stickstoff gespült. Anschließend wurde der Autoklav auf 280°C erhitzt. Nach einer Verweilzeit von 3 Stunden wurde der Autoklaveninhalt langsam ohne Abkühlung direkt in einen Wirbelofen, der mit 1000 g Al₂O₃ (Puralox, s. Bsp. 1) gefüllt war, entspannt. Das Al₂O₃ wurde mit Stickstoff am Wirbel gehalten. Die Temperatur in der Wirbelschicht betrug 300°C. Das die Wirbelschicht verlassende Dämpfegemisch wurde kondensiert. Das Kondensat enthielt 280 g Caprolactam.

## Patentansprüche

1. Verfahren zur Rückgewinnung von Caprolactam aus Oligomeren und/oder Polymeren des Caprolactams, welches folgende Schritte umfaßt:
a) Behandlung von Oligomeren und/oder Polymeren von Caprolactam mit 1 bis 20 Gew.-Teilen Wasser je Gew.-Teil Oligo- und/oder Polymer bei einer Temperatur von 200 bis 350°C unter erhöhtem Druck mit einer Verweilzeit von 0,5 bis 10 Stunden unter Bildung eines wäßrigen Reaktionsgemisches, das Polycaprolactam, monomeres Caprolactam und dessen Oligomere enthält und
b) Leiten des in a) erhaltenen wäßrigen Reaktionsgemisches in eine Aluminiumoxid-Wirbelschicht bei einer Temperatur von 250 bis 400°C unter Erhalt eines Gemisches aus Wasserdampf und Caprolactam.

2. Verfahren nach Anspruch 1, dadurch gekennzeichnet, daß man 2 bis 10 Gew.-Teile Wasser je Gew.-Teil Oligo- und/oder Polymer anwendet.

3. Verfahren nach den Ansprüchen 1 und 2, dadurch gekennzeichnet, daß man in Stufe a) eine Temperatur von 250 bis 300°C einhält.

4. Verfahren nach den Ansprüchen 1 bis 3, dadurch gekennzeichnet, daß man in der Stufe a) einen Druck von 15 bis 200 bar einhält.

5. Verfahren nach den Ansprüchen 1 bis 4, dadurch gekennzeichnet, daß man in der Stufe a) zusätzlich Phosphorsäure oder Natriumhydroxid mitverwendet.

6. Verfahren nach den Ansprüchen 1 bis 5, dadurch gekennzeichnet, daß man in der Stufe b) eine Temperatur von 290 bis 360°C einhält.

7. Verfahren nach den Ansprüchen 1 bis 6, dadurch gekennzeichnet, daß man eine γ-Aluminiumoxid-Wirbelschicht verwendet.

8. Verfahren nach den Ansprüchen 1 bis 7, dadurch gekennzeichnet, daß man in der Wirbelschicht eine Verweilzeit von 0,1 bis 30 Sekunden einhält.

9. Verfahren nach den Ansprüchen 1 bis 8, dadurch gekennzeichnet, daß man das wäßrige Reaktionsgemisch aus der Stufe a) direkt über eine Düsenöffnung in die Wirbelschicht der Stufe b) entspannt.
